# EUROPEAN PATENT APPLICATION

(11) **EP 4 778 545 A1**
(43) Date of publication of application: **22.07.2026**
(21) Application number: 24874680.2
(22) Date of filing: 03.10.2024
(51) Int. Cl.: A61M 1/16, A61M 1/36

(54) **BLOOD PURIFICATION DEVICE**

(30) Priority: 06.10.2023 JP 2023174754
(71) Applicant: Nikkiso Company Limited, Tokyo 150-6022 (JP)
(72) Inventor: KAWARABAYASHI, Satoru, Tokyo 150-6022 (JP); HASEGAWA, Shinya, Higashimurayama-shi, Tokyo 189-8520 (JP); AKITA, Kunihiko, Higashimurayama-shi, Tokyo 189-8520 (JP)
(74) Representative: von Hirschhausen, Helge
(86) International application number: PCT/JP2024/035417
(87) International publication number: WO 2025/075080

(57) **Abstract**

Provided is a blood purification apparatus that achieves a reduction in the amount of priming solution to be administered to a patient together with an anticoagulant before a blood purification treatment. A blood purification apparatus includes a control unit configured to control a blood pump 4 and an infusion pump 5. The control unit 9 is configured to activate the infusion pump 5 by supplying a driving signal to the infusion pump 5 in a state where a blood circuit is filled with a priming solution; cause the blood pump 4 to undergo a first operation by supplying a first driving signal to the blood pump 4 in a state where an anticoagulant is infused into the blood circuit by the activation of the infusion pump 5; and supply a second driving signal to the blood pump 4 in a state where the anticoagulant is transferred to a distal end of an arterial blood circuit 1 and/or a distal end of a venous blood circuit 2 by the first operation of the blood pump 4 and where a patient is punctured with the distal end of the arterial blood circuit 1 and the distal end of the venous blood circuit 2.

## Description

### Technical Field

The present invention relates to a blood purification apparatus configured to purify a patient's blood by causing the blood to extracorporeally circulate through a blood circuit, including an arterial blood circuit and a venous blood circuit, and a blood purifier.

### Background Art

A dialysis apparatus serving as a blood purification apparatus intended for dialysis treatment and the like typically includes a line section including a dialysate introduction line for introducing dialysate into a blood purifier and a drain-liquid discharge line for discharging drain liquid from the blood purifier, and a liquid-delivery section for delivering the dialysate and the drain liquid in the line section. The dialysis apparatus is obtained by connecting to the blood purifier a blood circuit for causing a patient's blood to extracorporeally circulate. The dialysis apparatus is configured to perform dialysis treatment (blood purification treatment) at the blood purifier while the patient's blood is caused to extracorporeally circulate through the blood circuit.

On the other hand, to avoid blood coagulation, an anticoagulant needs to be administered to the patient before the patient's blood is caused to extracorporeally circulate in the blood purification treatment. To enable the anticoagulant to satisfactorily exert its effect of suppressing blood coagulation, the blood purification treatment needs to be executed after the elapse of a predetermined time (about 3 to 5 minutes, for example) from the administration of the anticoagulant to the patient. Accordingly, as disclosed in PTL 1, a blood purification apparatus hitherto proposed is configured to execute blood purification treatment after the elapse of a predetermined time from when an anticoagulant infused into a blood circuit in advance is administered into a patient's body together with a priming solution.

### Citation List

### Patent Literature

PTL 1: Japanese Unexamined Patent Application Publication No. 2020-14851

### Summary of Invention

### Technical Problem

In the above hitherto proposed blood purification apparatus, however, if, for example, the flow route to be used in administering the anticoagulant into the patient's body has a relatively long length from the position of infusion of the anticoagulant to the patient, an excessively large amount of priming solution that corresponds to the priming volume of such a flow route is administered into the patient's body together with the anticoagulant. Patients before having blood purification treatment are typically in a state of fluid overload. Therefore, it is not desirable for a patient in the state of fluid overload to be administered an excessively large amount of priming solution.

The present invention has been conceived in view of the above circumstances and provides a blood purification apparatus that achieves a reduction in the amount of priming solution to be administered to a patient together with an anticoagulant before a blood purification treatment.

### Solution to Problem

A blood purification apparatus as an embodiment according to the present invention is a blood purification apparatus configured to purify a patient's blood by causing the blood to extracorporeally circulate through a blood circuit and a blood purifier, the blood circuit including an arterial blood circuit and a venous blood circuit. The blood purification apparatus includes a blood pump configured to cause the blood in the blood circuit to flow for extracorporeal circulation, an infusion pump configured to infuse an anticoagulant into a predetermined site, and a control unit configured to control the blood pump and the infusion pump. The control unit is configured to activate the infusion pump by supplying a driving signal to the infusion pump in a state where the blood circuit is filled with a priming solution, cause the blood pump to undergo a first operation by supplying a first driving signal to the blood pump in a state where the anticoagulant is infused into the blood circuit by the activation of the infusion pump, and supply a second driving signal to the blood pump in a state where the anticoagulant is transferred to a distal end of the arterial blood circuit and/or a distal end of the venous blood circuit by the first operation of the blood pump and where the patient is punctured with the distal end of the arterial blood circuit and the distal end of the venous blood circuit.

### Advantageous Effects of Invention

According to the present invention, the infusion pump is activated by supplying a driving signal to the infusion pump in a state where the blood circuit is filled with a priming solution, the blood pump is caused to undergo a first operation by supplying a first driving signal to the blood pump in a state where the anticoagulant is infused into the blood circuit by the activation of the infusion pump, and a second driving signal is supplied to the blood pump in a state where the anticoagulant is transferred to a distal end of the arterial blood circuit and/or a distal end of the venous blood circuit by the first operation of the blood pump and where the patient is punctured with the distal end of the arterial blood circuit and the distal end of the venous blood circuit. Thus, a reduction in the amount of priming solution to be administered to the patient together with the anticoagulant before the blood purification treatment is achieved.

### Brief Description of Drawings

[Fig. 1] Fig. 1 is a diagram illustrating a blood purification apparatus according to an embodiment of the present invention.
[Fig. 2] Fig. 2 is a flow chart illustrating an entire process to be performed by the blood purification apparatus.
[Fig. 3] Fig. 3 is a flow chart illustrating an administration preparation step to be performed by the blood purification apparatus.
[Fig. 4] Fig. 4 is a diagram illustrating a state of the blood purification apparatus where a priming step is underway.
[Fig. 5] Fig. 5 is a diagram illustrating a state of the blood purification apparatus where an infusion step is underway.
[Fig. 6] Fig. 6 is a diagram illustrating a state of the blood purification apparatus where a transfer step (transfer to only the distal end of a venous blood circuit) is underway.
[Fig. 7] Fig. 7 is a diagram illustrating a state of the blood purification apparatus with puncture needles attached thereto.
[Fig. 8] Fig. 8 is a diagram illustrating a state of the blood purification apparatus where an administration step is underway.
[Fig. 9] Fig. 9 is a diagram illustrating a state of the blood purification apparatus where a blood-purification-treatment step is underway.
[Fig. 10] Fig. 10 is a block diagram illustrating a control unit and peripheral elements included in the blood purification apparatus.
[Fig. 11] Fig. 11 is a diagram illustrating a state of the blood purification apparatus where a transfer step (transfer to only the distal end of an arterial blood circuit) is underway.
[Fig. 12] Fig. 12 is a diagram illustrating a state of the blood purification apparatus where a transfer step (transfer to the distal ends of the arterial blood circuit and the venous blood circuit) is underway.
[Fig. 13] Fig. 13 is a time chart illustrating operations of the peripheral elements of the control unit included in the blood purification apparatus (in the case of transfer to only the distal end of the venous blood circuit).
[Fig. 14] Fig. 14 is a time chart illustrating operations of the peripheral elements of the control unit included in the blood purification apparatus (in the case of transfer to only the distal end of the arterial blood circuit).
[Fig. 15] Fig. 15 is a time chart illustrating operations of the peripheral elements of the control unit included in the blood purification apparatus (in the case of transfer to the distal ends of the arterial blood circuit and the venous blood circuit).
[Fig. 16] Fig. 16 is a diagram illustrating a blood purification apparatus according to another embodiment of the present invention.

### Description of Embodiments

Embodiments of the present invention will now be described specifically with reference to the drawings.

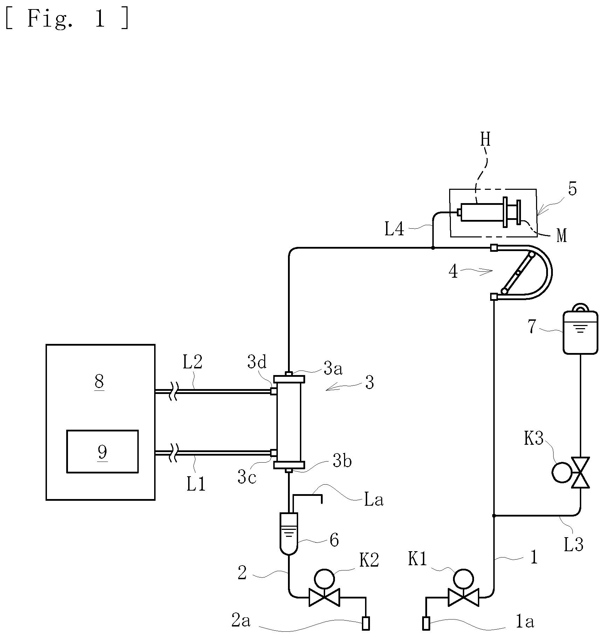

A blood purification apparatus according to the present embodiment is configured to purify a patient's blood by causing the blood to extracorporeally circulate through a blood circuit, including an arterial blood circuit and a venous blood circuit, and a blood purifier. As illustrated in Fig. 1, the blood purification apparatus includes a blood circuit including an arterial blood circuit 1 and a venous blood circuit 2, a dialyzer 3 serving as a blood purifier, a blood pump 4, an infusion pump 5, a dialysis device 8 capable of supplying dialysate to the dialyzer 3, and a control unit 9.

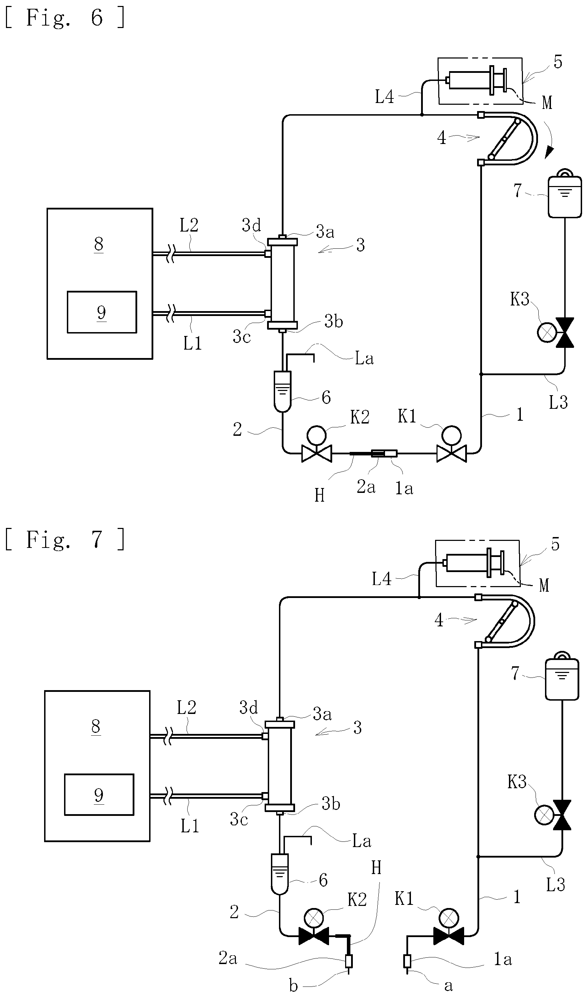

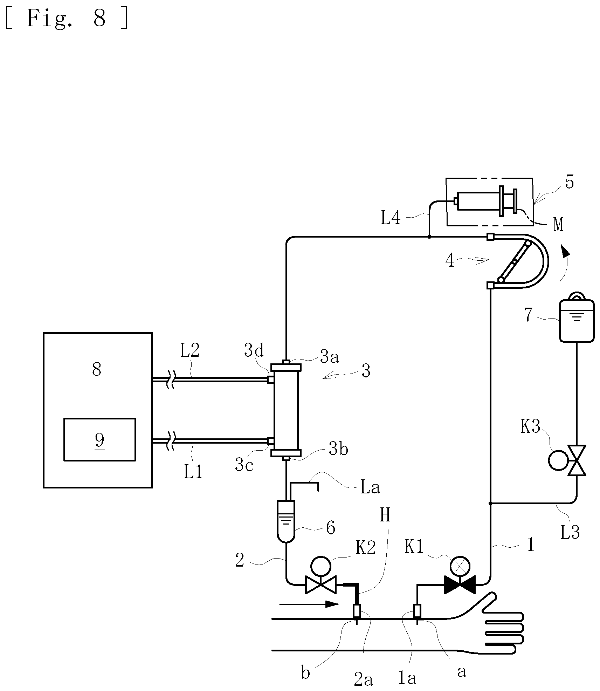

The arterial blood circuit 1 is provided with a connector 1a at the distal end thereof. An arterial puncture needle a (see Figs. 7 and 8) is connectable to the arterial blood circuit 1 via the connector 1a. A peristaltic blood pump 4 is provided at a halfway point of the arterial blood circuit 1. The blood pump 4 is a pump provided at a predetermined position of the arterial blood circuit 1 and is intended to cause the blood and a priming solution in the blood circuit to flow when activated.

On the other hand, the venous blood circuit 2 is provided with a connector 2a at the distal end thereof. A venous puncture needle b (see Figs. 7 and 8) is connectable to the venous blood circuit 2 via the connector 2a. An air-trap chamber 6 is provided at a halfway point of the venous blood circuit 2. The air-trap chamber 6 is a chamber connected to a predetermined position of the venous blood circuit 2 and is intended to trap and remove air contained in the blood that is under extracorporeal circulation.

A distal portion of the arterial blood circuit 1 and a distal portion of the venous blood circuit 2 are provided with respective clamping means K1 and K2, which are electromagnetic valves. Opening the clamping means K1 or K2 opens the flow route at the distal portion of the arterial blood circuit 1 or the venous blood circuit 2. Closing the clamping means K1 or K2 closes the flow route at the distal portion of the arterial blood circuit 1 or the venous blood circuit 2.

The dialyzer 3 (blood purifier) has, in a housing thereof, a blood inlet 3a (blood introduction port), a blood outlet 3b (blood delivery port), a dialysate inlet 3c (dialysate introduction port), and a dialysate outlet 3d (dialysate delivery port). The arterial blood circuit 1 is connected to the blood inlet 3a. The venous blood circuit 2 is connected to the blood outlet 3b. The dialysate inlet 3c and the dialysate outlet 3d are connected to a dialysate introduction line L1 and a drain-liquid discharge line L2, respectively.

The dialyzer 3 houses a plurality of hollow fiber membranes (not illustrated), which constitute blood purification membranes for purifying blood. The hollow fiber membranes constituting the blood purification membranes each have a number of microscopic holes (pores) extending therethrough from the outer surface to the inner surface. Impurities and the like contained in the blood are allowed to permeate through the hollow fiber membranes into the dialysate, whereby purification is achieved.

The dialysate introduction line L1, through which the dialysate is introduced into the dialyzer 3, and the drain-liquid discharge line L2, through which the drain liquid is discharged from the dialyzer 3, extend from the dialysis device 8. The dialysate introduction line L1 is connected at one end thereof to the dialyzer 3 (dialysate inlet 3c) and at the other end thereof to dialysate-making means (not illustrated) configured to prepare a dialysate at a predetermined concentration. Accordingly, the dialysate at a predetermined concentration is allowed to be introduced into the dialyzer 3.

The drain-liquid discharge line L2 is connected at one end thereof to the dialyzer 3 (dialysate outlet 3d) and at the other end thereof to draining means, which is not illustrated. Accordingly, after the dialysate supplied from a dialysate supply device flows through the dialysate introduction line L1 and reaches the dialyzer 3, the drain liquid from the dialyzer 3 flows through the drain-liquid discharge line L2 and is delivered to the draining means. The dialysis device 8 is provided with a liquid delivery pump for causing the dialysate and the drain liquid to flow, and an ultrafiltration pump (not illustrated) for removing water from the patient's blood flowing in the dialyzer 3.

When the blood pump 4 is activated in a state where the patient is punctured with the arterial puncture needle a connected to the distal end of the arterial blood circuit 1 and the venous puncture needle b connected to the distal end of the venous blood circuit 2, the patient's blood flows through the arterial blood circuit 1 and reaches the dialyzer 3, where the blood is subjected to a blood purification treatment. Then, the blood flows through the venous blood circuit 2 and returns into the patient's body. Thus, the patient's blood can be purified at the dialyzer 3 while being caused to extracorporeally circulate through the blood circuit.

The arterial blood circuit 1 according to the present embodiment is provided with a physiological saline line L3 at a position between the connector 1a and the site where the blood pump 4 is provided. A container 7 is connected to the distal end of the physiological saline line L3. The container 7 contains a predetermined amount of priming solution (physiological saline). The physiological saline line L3 is provided with clamping means K3, which is an electromagnetic valve. Opening the clamping means K3 opens the physiological saline line L3, whereby the priming solution in the container 7 is allowed to be supplied to the blood circuit. Closing the clamping means K3 closes the physiological saline line L3, whereby the supply of the priming solution toward the blood circuit is stopped.

The arterial blood circuit 1 according to the present embodiment is further provided with an anticoagulant infusion line L4 between the site where the blood pump 4 is provided and the site where the arterial blood circuit 1 is connected to the dialyzer 3. A syringe M is connected to the distal end of the anticoagulant infusion line L4. The syringe M contains a predetermined amount of anticoagulant (heparin H, in the present embodiment). The syringe M is attached to the infusion pump 5, which is included in the dialysis device 8. The syringe M is capable of infusing a predetermined amount of anticoagulant contained therein, with a pusher (not illustrated) included in the infusion pump 5 causing the plunger of the syringe M to slide.

The heparin H serving as an anticoagulant is composed of, for example, unfractionated heparin, low-molecular-weight heparin, and the like. The heparin H is initially administered before the blood purification treatment (dialysis treatment). According to need, the heparin H is also administered continuously during the blood purification treatment (continuous administration). Instead of heparin, an anticoagulant of any other kind that can exert an effect of suppressing coagulation may be infused and administered.

In the present embodiment, the capacity between the connected part of the anticoagulant infusion line L4 and the distal end of the arterial blood circuit 1 is 45.1 (ml), and the flow rate of the blood pump 4 is 8.696 (ml/revolution). Under such conditions, reversely rotating the blood pump 4 by about six revolutions (52.2 (ml)) in a transfer step, to be described below, transfers the heparin H from the anticoagulant infusion line L4 to a position near the distal end of the venous blood circuit 2; and normally rotating the blood pump 4 by about two revolutions (17.4 (ml)) after the connection to the patient in an administration step, to be described below, administers the heparin H from the venous blood circuit 2 to the patient. Note that the capacity of the blood circuit varies with the type of the apparatus and needs to be confirmed in advance and set in the apparatus accordingly. The numbers of revolutions required and to be adjusted in the transfer step and the administration step need to be verified in advance through an experiment for finding how much heparin H is diffused in the transfer step.

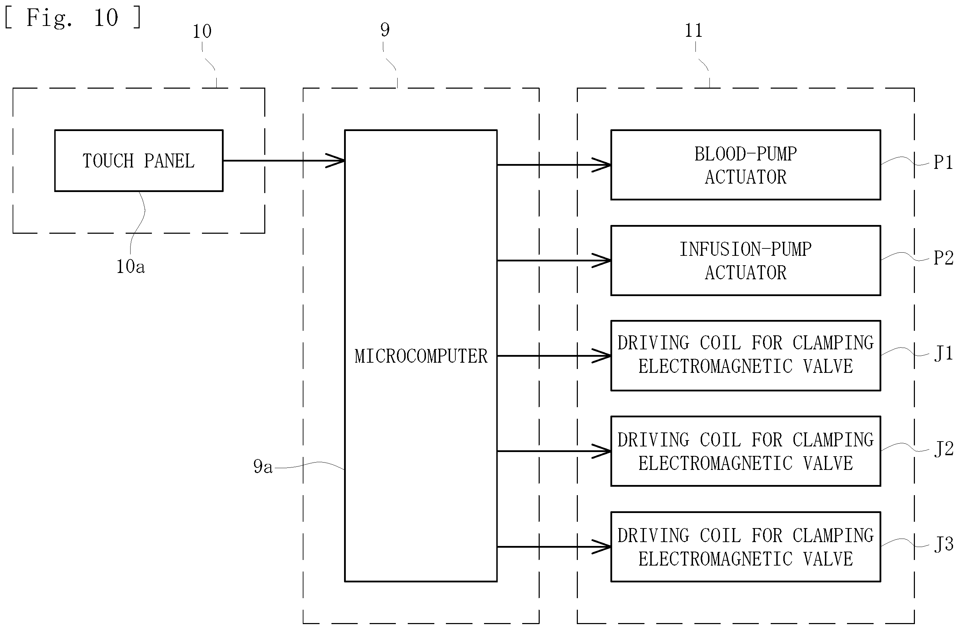

The control unit 9 is a microcomputer or the like (specifically, a microcomputer 9a illustrated in Fig. 10) and is included in the dialysis device 8. The control unit 9 is electrically connected to the blood pump 4, the infusion pump 5, and the clamping means (K1 to K3). The control unit 9 is configured to control the activation of the blood pump 4 and the infusion pump 5 and the operations of the clamping means (K1 to K3), thereby being capable of executing a priming step of filling the blood circuit with the priming solution and a blood-purification-treatment step of purifying the patient's blood at the dialyzer 3 while causing the blood to extracorporeally circulate through the blood circuit.

The control unit 9 according to the present embodiment is capable of sequentially executing the following after the priming step: an infusion step of infusing the anticoagulant (heparin H) into the blood circuit by activating the infusion pump 5, the transfer step of transferring the heparin H infused in the infusion step to the distal end of the arterial blood circuit 1 and/or the distal end of the venous blood circuit 2 by activating the blood pump 4, the administration step of administering the heparin H transferred in the transfer step to the patient by activating the blood pump 4 in a state where the patient is punctured with the distal end of the arterial blood circuit 1 and the distal end of the venous blood circuit 2, and a standby step of standing by after the administration step until a predetermined time elapses.

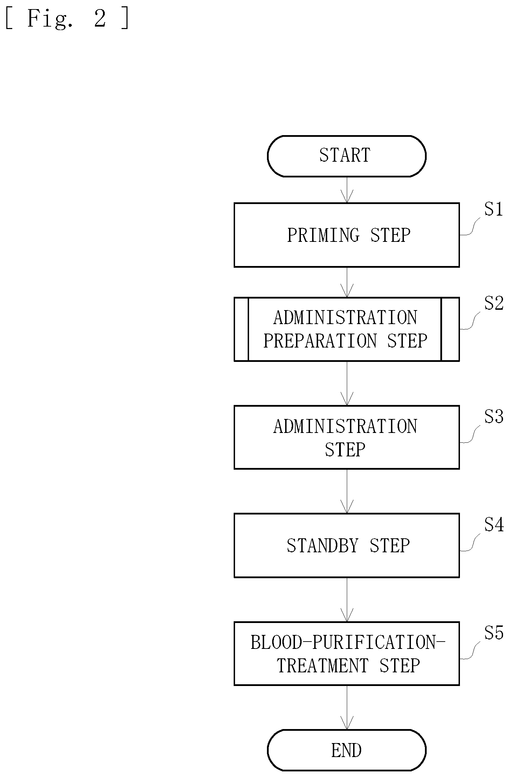

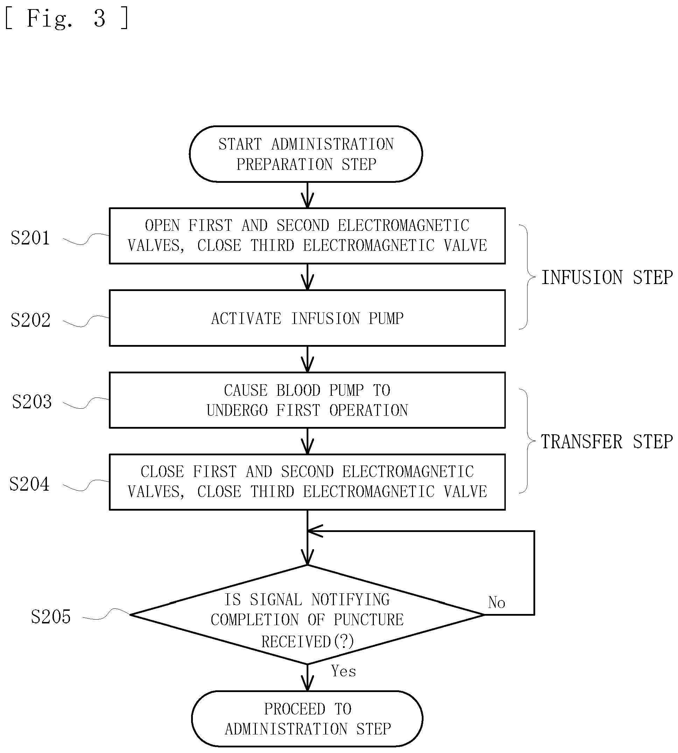

Now, a control process to be performed by the control unit 9 according to the present embodiment will be described with reference to the flow charts illustrated in Figs. 2 and 3.

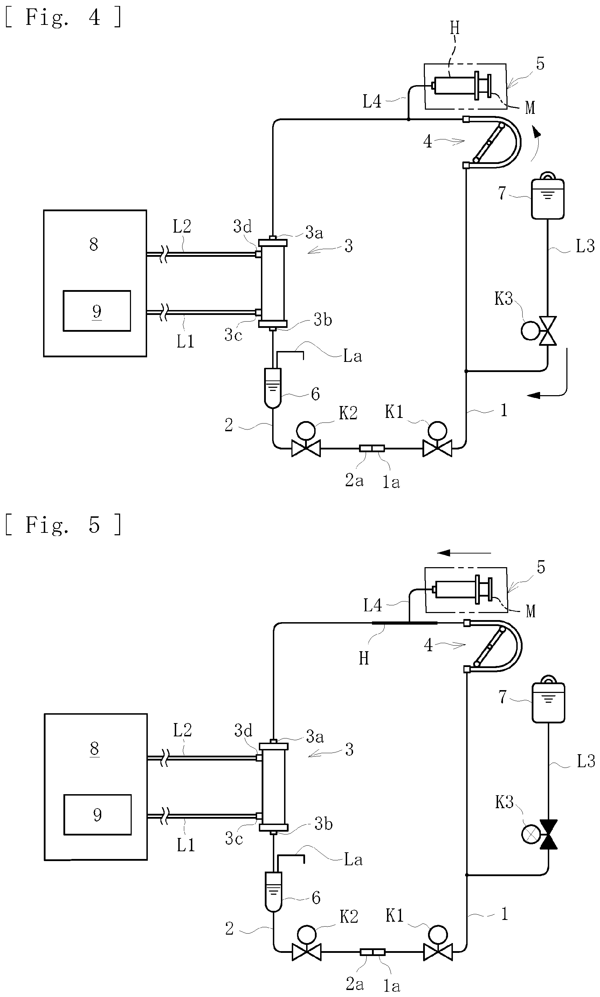

First, as illustrated in Fig. 4, the connector 1a at the distal end of the arterial blood circuit 1 and the connector 2a at the distal end of the venous blood circuit 2 are connected to each other to form a closed circuit. Then, the blood pump 4 is activated (normally rotated) with the clamping means K3 open. Thus, a priming step S1 is executed in which the priming solution (physiological saline) in the container 7 is introduced into the blood circuit and fills the blood circuit. In the priming step S1, the priming solution in the container 7 circulates within the closed circuit formed by the arterial blood circuit 1 and the venous blood circuit 2 and is discharged to the outside through an overflow line La, which extends from the top of the air-trap chamber 6. Thus, the closed circuit is filled with the priming solution. Note that the priming method is not limited to the above. The priming step may be based on any of various priming methods.

After the priming step S1, the process proceeds to an administration preparation step S2. The administration preparation step S2 includes the following. As illustrated in Fig. 3, in step S201, the first electromagnetic valve and the second electromagnetic valve (clamping means K1 and K2) are opened, whereas the third electromagnetic valve (clamping means K3) is closed. In step S202, a driving signal is transmitted to an actuator P2 for the infusion pump 5 to activate the infusion pump 5 (Fig. 5). Subsequently, in step S203, a first driving signal is transmitted to an actuator P1 for the blood pump 4. Accordingly, as illustrated in Fig. 6, the blood pump 4 is activated to transfer the heparin H (the transfer step). Furthermore, in step S204, the first and second electromagnetic valves (clamping means K1 and K2) are closed, and the third electromagnetic valve (clamping means K3) is kept closed (see Fig. 7). Furthermore, in step S205, it is determined whether a signal notifying the completion of puncture with the distal end of the arterial blood circuit 1 and the distal end of the venous blood circuit 2 is received.

If it is determined in step S205 that the signal notifying the completion of puncture with the distal end of the arterial blood circuit 1 and the distal end of the venous blood circuit 2 is received, the process proceeds to an administration step S3. In the administration step S3, as illustrated in Fig. 8, in a state where the patient is punctured with the arterial puncture needle a and the venous puncture needle b, the blood pump 4 is normally rotated with the clamping means K1 closed, the clamping means K2 open, and the clamping means K3 open. Accordingly, the heparin H at the distal portion (the portion near the connector 2a) of the venous blood circuit 2 is administered into the patient's body through the venous puncture needle b. Note that the amount of heparin H to be administered in the administration step S3 can be estimated from the number of revolutions of the blood pump 4.

After the administration step S3, a standby step S4 is executed, in which the blood pump 4 and the infusion pump 5 are stopped for standby until a predetermined time (about 3 to 5 minutes, for example) elapses. Standing by until a predetermined time elapses in the standby step S4 activates the heparin H administered into the patient's body to satisfactorily increase the effect of suppressing blood coagulation, and therefore suppresses the coagulation of the patient's blood that is thereafter caused to extracorporeally circulate in a blood-purification-treatment step S5.

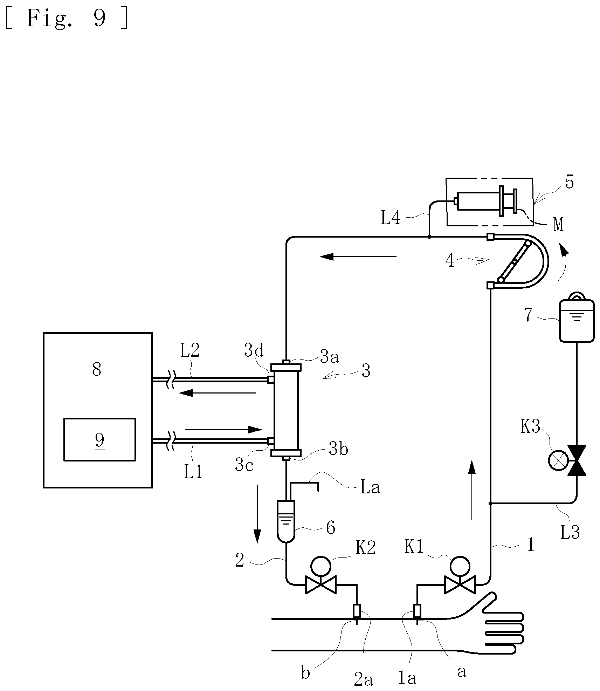

After the standby step S4, as illustrated in Fig. 9, with the patient kept punctured with the arterial puncture needle a and the venous puncture needle b, the blood pump 4 is normally rotated. Simultaneously, the dialysate is introduced into the dialyzer 3 through the dialysate introduction line L1, meanwhile the drain liquid is discharged through the drain-liquid discharge line L2. Thus, the blood-purification-treatment step S5 is executed, in which the patient's blood is purified at the dialyzer 3 while being caused to extracorporeally circulate through the blood circuit.

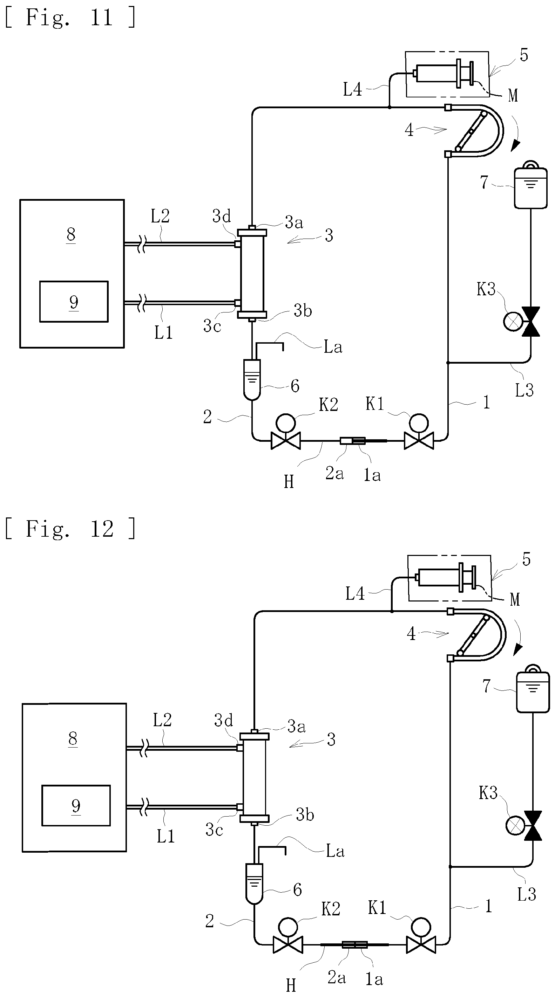

In the transfer step according to the present embodiment, as illustrated in Fig. 6, the heparin H is transferred to only the distal end of the venous blood circuit 2. Alternatively, the heparin H may be transferred to only the distal end of the arterial blood circuit 1 as illustrated in Fig. 11, or to both the distal end of the arterial blood circuit 1 and the distal end of the venous blood circuit 2 as illustrated in Fig. 12.

Now, peripheral elements of the control unit 9 according to the present embodiment and control operations of the peripheral elements will be described with reference to the block diagram illustrated in Fig. 10.

As illustrated in Fig. 10, the control unit 9 according to the present embodiment includes the microcomputer 9a. The microcomputer 9a is electrically connected to a touch panel 10a of an input unit 10 so as to be capable of receiving an input signal from the touch panel 10a. The microcomputer 9a is connected to a drive unit 11. The drive unit 11 includes the actuator P1 for the blood pump 4, the actuator P2 for the infusion pump 5, a driving coil J1 for the clamping means K1 (first electromagnetic valve), a driving coil J2 for the clamping means K2 (second electromagnetic valve), and a driving coil J3 for the clamping means K3 (third electromagnetic valve).

In the present embodiment, the microcomputer 9a of the control unit 9 is configured to execute a process for activating the infusion pump 5 by supplying a driving signal to the actuator P2 for the infusion pump 5 in a state where the blood circuit is filled with the priming solution, causing the blood pump 4 to undergo a first operation by supplying a first driving signal to the actuator P1 for the blood pump 4 in a state where the anticoagulant is infused into the blood circuit by the activation of the infusion pump 5, and causing the blood pump 4 to undergo a second operation by supplying a second driving signal to the actuator P1 for the blood pump 4 in a state where the anticoagulant is transferred to the distal end of the arterial blood circuit 1 or the distal end of the venous blood circuit 2 by the first operation of the blood pump 4 and where the patient is punctured with the distal end of the arterial blood circuit 1 and the distal end of the venous blood circuit 2.

Furthermore, the present embodiment employs the first electromagnetic valve (clamping means K1) configured to open and close an end portion of the arterial blood circuit 1, the second electromagnetic valve (clamping means K2) configured to open and close an end portion of the venous blood circuit 2, and the third electromagnetic valve (clamping means K3) configured to open and close a supply line through which dialysate is to be supplied. The microcomputer 9a of the control unit 9 is configured to execute a process for opening the first electromagnetic valve and the second electromagnetic valve and closing the third electromagnetic valve before the anticoagulant is infused, closing the first electromagnetic valve and the second electromagnetic valve after the anticoagulant is transferred to the distal end of the arterial blood circuit 1 or the distal end of the venous blood circuit 2, and closing the third electromagnetic valve.

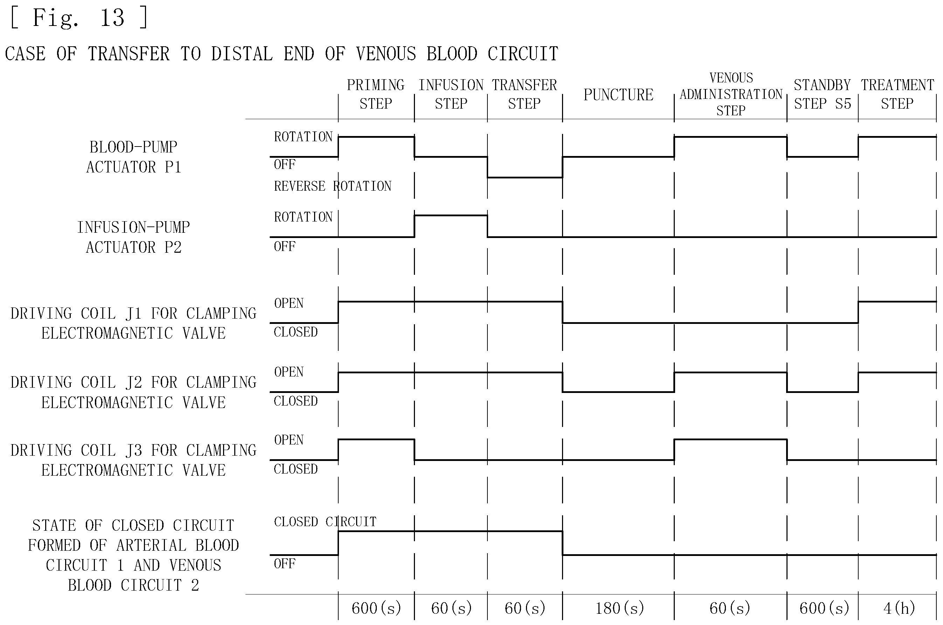

Now, the control unit 9 and the peripheral elements according to the present embodiment and control operations of the peripheral elements will be described with reference to the timing chart illustrated in Fig. 13. The present timing chart assumes that the heparin H is transferred to only the distal end of the venous blood circuit 2 (see Fig. 6).

In the priming step (600 (s)), the blood-pump actuator P1 is on (with a driving signal for normal rotation) to cause the blood pump 4 to undergo normal rotation, whereas the infusion-pump actuator P2 is off (with no driving signal) to stop the infusion pump 5. Furthermore, the driving coil J1 for the clamping electromagnetic valve, the driving coil J2 for the clamping electromagnetic valve, and the driving coil J3 for the clamping electromagnetic valve are on (with driving signals), whereby the flow routes are open. While the present embodiment relates to a case where the clamping electromagnetic valves (J1 to J3) are closed in the initial state (a state with no driving signals), the electromagnetic valves may be open in the initial state.

In the infusion step (60 (s)), the blood-pump actuator P1 is off (with no driving signal) to stop the blood pump 4, whereas the infusion-pump actuator P2 is on (with a driving signal) to actuate the infusion pump 5. Furthermore, the driving coil J1 for the clamping electromagnetic valve and the driving coil J2 for the clamping electromagnetic valve are on (with driving signals) to open the flow routes, whereas the driving coil J3 for the clamping electromagnetic valve is off (with no driving signal) to close the flow route.

In the transfer step (60 (s)), the blood-pump actuator P1 is on (with a driving signal for reverse rotation) to cause the blood pump 4 to undergo reverse rotation, whereas the infusion-pump actuator P2 is off (with no driving signal) to stop the infusion pump 5. Furthermore, the driving coil J1 for the clamping electromagnetic valve and the driving coil J2 for the clamping electromagnetic valve are on (with driving signals) to open the flow routes, whereas the driving coil J3 for the clamping electromagnetic valve is off (with no driving signal) to close the flow route.

During puncture work that is performed thereafter, the blood-pump actuator P1 is off (with no driving signal) to stop the blood pump 4, and the infusion-pump actuator P2 is off (with no driving signal) to stop the infusion pump 5. Furthermore, the driving coil J1 for the clamping electromagnetic valve, the driving coil J2 for the clamping electromagnetic valve, and the driving coil J3 for the clamping electromagnetic valve are off (with no driving signals) to close the flow routes.

In a venous administration step (60 (s)), the blood-pump actuator P1 is on (with a driving signal for normal rotation) to cause the blood pump 4 to undergo normal rotation, whereas the infusion-pump actuator P2 is off (with no driving signal) to stop the infusion pump 5. Furthermore, the driving coil J2 for the clamping electromagnetic valve and the driving coil J3 for the clamping electromagnetic valve are on (with driving signals) to open the flow routes, whereas the driving coil J1 for the clamping electromagnetic valve is off (with no driving signal) to close the flow route.

In the standby step (600 (s)), the blood-pump actuator P1 is off (with no driving signal) to stop the blood pump 4, and the infusion-pump actuator P2 is off (with no driving signal) to stop the infusion pump 5. Furthermore, the driving coil J1 for the clamping electromagnetic valve, the driving coil J2 for the clamping electromagnetic valve, and the driving coil J3 for the clamping electromagnetic valve are off (with no driving signals) to close the flow routes.

At the start of the blood-purification-treatment step (4 (h)), the blood-pump actuator P1 is on (with a driving signal for normal rotation) to cause the blood pump 4 to undergo normal rotation, whereas the infusion-pump actuator P2 is off (with no driving signal) to stop the infusion pump 5. Furthermore, the driving coil J1 for the clamping electromagnetic valve and the driving coil J2 for the clamping electromagnetic valve are on (with driving signals) to open the flow routes, whereas the driving coil J3 for the clamping electromagnetic valve is off (with no driving signal) to close the flow route.

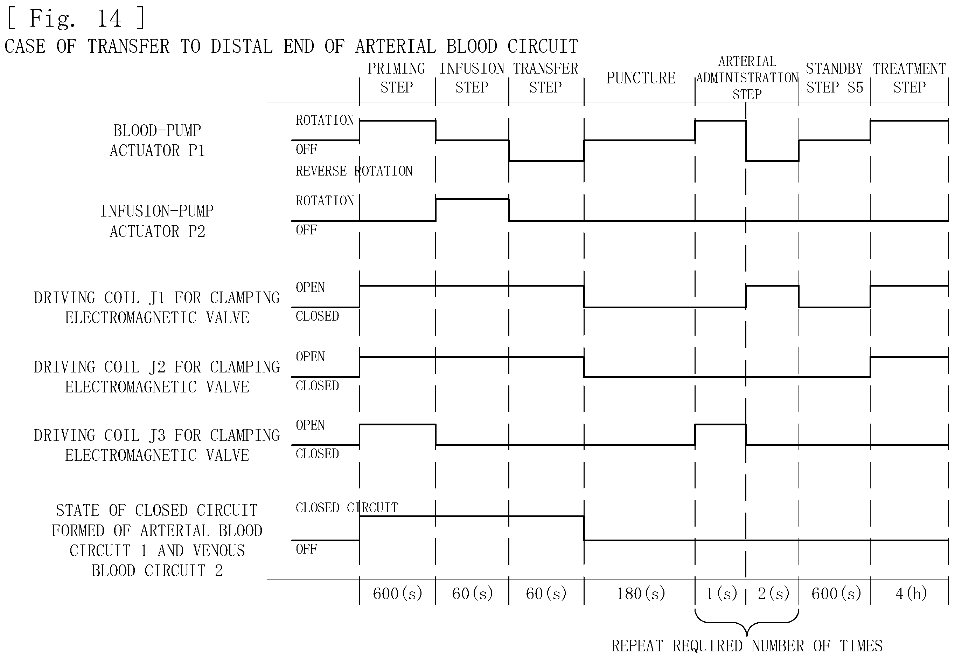

Now, the control unit 9 the peripheral elements according to the present embodiment and control operations of the peripheral elements will be described with reference to the timing chart illustrated in Fig. 14. The present timing chart assumes that the heparin H is transferred to only the distal end of the arterial blood circuit 1 (see Fig. 11). The priming step, the infusion step, the transfer step, the puncture step, the standby step, and the treatment step are the same as in the case where the heparin H is transferred to only the distal end of the venous blood circuit 2.

In an arterial administration step (1 (s) and 2 (s)), the following states are repeatedly established a required number of times: a state where the blood-pump actuator P1 is on (with a driving signal for normal rotation) to cause the blood pump 4 to undergo normal rotation, the infusion-pump actuator P2 is off (with no driving signal) to stop the infusion pump 5, the driving coil J3 for the clamping electromagnetic valve is on (with a driving signal) to open the flow route, and the driving coil J1 for the clamping electromagnetic valve and the driving coil J2 for the clamping electromagnetic valve are off (with no driving signals) to close the flow routes; and a state where the blood-pump actuator P1 is on (with a driving signal for reverse rotation) to cause the blood pump 4 to undergo reverse rotation, the infusion-pump actuator P2 is off (with no driving signal) to stop the infusion pump 5, the driving coil J1 for the clamping electromagnetic valve is on (with a driving signal) to open the flow route, and the driving coil J2 for the clamping electromagnetic valve and the driving coil J3 for the clamping electromagnetic valve are off (with no driving signals) to close the flow routes.

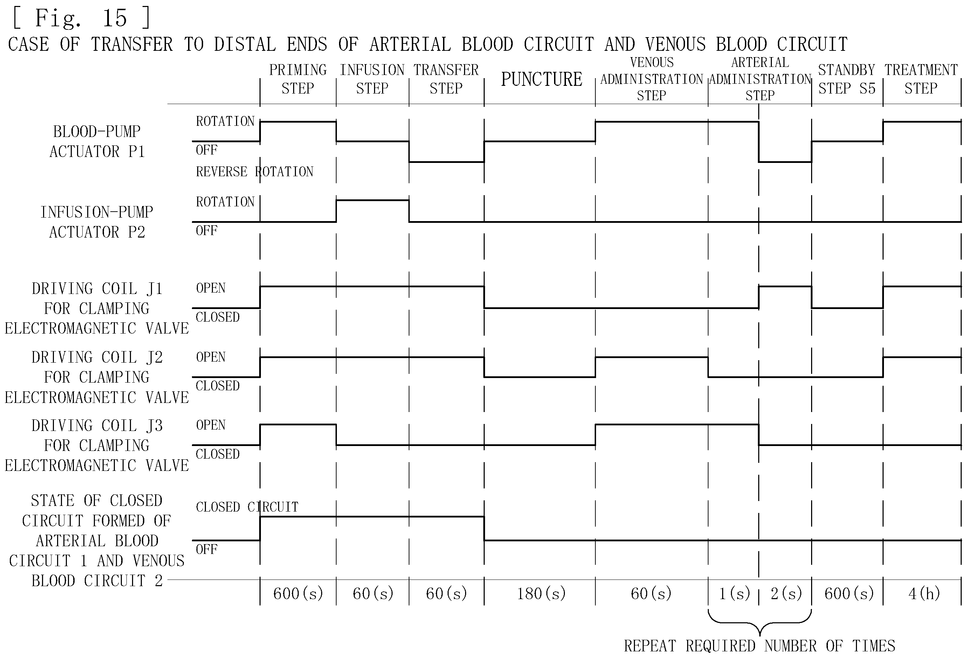

Now, the control unit 9 and the peripheral elements according to the present embodiment and control operations of the peripheral elements will be described with reference to the timing chart illustrated in Fig. 15. The present timing chart assumes that the heparin H is transferred to the distal ends of the arterial blood circuit 1 and the venous blood circuit 2 (see Fig. 12). The priming step, the infusion step, the transfer step, the puncture step, the standby step, the venous administration step, and the treatment step are the same as in the case where the heparin H is transferred to only the distal end of the venous blood circuit 2. The arterial administration step is the same as in the case where the heparin H is transferred to only the distal end of the arterial blood circuit 1.

According to the present embodiment, after the priming step S1, the control unit 9 sequentially executes the following: the infusion step of infusing the anticoagulant into the blood circuit by activating the infusion pump 5, the transfer step of transferring the anticoagulant infused in the infusion step by causing the blood pump to undergo the first operation to the distal end of the arterial blood circuit 1 and the distal end of the venous blood circuit 2, and the administration step S3 of administering the anticoagulant transferred in the transfer step to the patient by causing the blood pump 4 to undergo the second operation in a state where the patient is punctured with the distal end of the arterial blood circuit 1 and the distal end of the venous blood circuit 2. Such a process reduces the amount of priming solution (physiological saline) to be administered to the patient together with the anticoagulant before the blood purification treatment.

Furthermore, the reduction in the amount of priming solution to be administered decreases the cardiac load due to fluid overload (prevents the increase in blood pressure). Moreover, an increase in the effect of suppressing blood coagulation enables a reduction in the amount of heparin to be administered, leading to a reduction in the cost of anticoagulant (heparin) and a reduction in side effects of the anticoagulant (heparin). Furthermore, while the known art makes medical workers measure the standby period and wait until the treatment starts, such a workload imposed on medical workers can be omitted with the automated operation of the apparatus.

The control unit 9 according to the present embodiment is configured to execute the standby step of standing by until a predetermined time elapses after the administration step. Thus, the heparin H administered into the patient's body is activated, whereby the effect of suppressing blood coagulation is satisfactorily increased. Consequently, the coagulation of the patient's blood to be caused to extracorporeally circulate thereafter in the blood-purification-treatment step S5 is suppressed.

In the priming step S1 according to the present embodiment, the distal end of the arterial blood circuit 1 and the distal end of the venous blood circuit 2 are connected to each other to form a closed circuit, and the closed circuit is filled with the priming solution (physiological saline). The closed circuit is maintained in the infusion step and in the transfer step. Thus, the closed circuit formed in the priming step S1 is utilized in executing the infusion step and the transfer step.

In the transfer step according to the present embodiment, the blood pump 4 is reversely rotated to transfer the heparin H to the distal end of the arterial blood circuit 1 and the distal end of the venous blood circuit 2. Thus, the heparin H infused in the infusion step is prevented from flowing through the dialyzer 3 and is assuredly transferred to the distal end of the arterial blood circuit 1 and the distal end of the venous blood circuit 2.

In the administration step S3 according to the present embodiment, the blood pump 4 is normally rotated to administer the heparin H at the distal portion of the venous blood circuit 2 to the patient, and the blood pump 4 is reversely rotated to administer the heparin H at the distal portion of the arterial blood circuit 1 to the patient. Thus, the heparin H at the distal portion of the venous blood circuit 2 and the heparin H at the distal portion of the arterial blood circuit 1 are each assuredly administered to the patient.

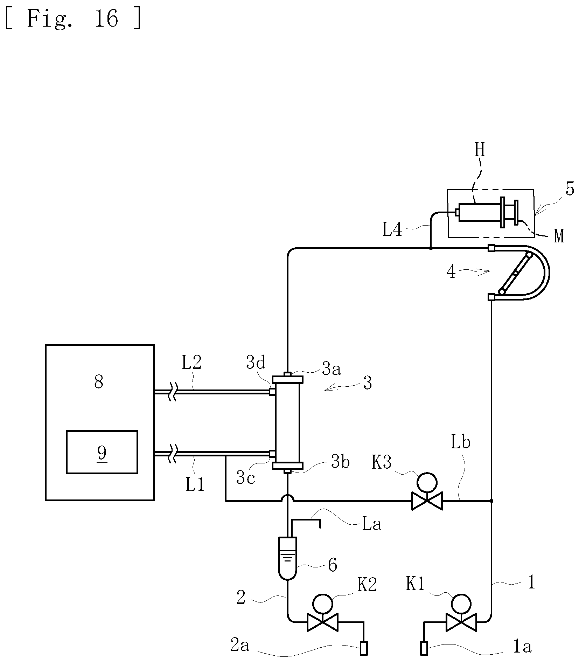

While an embodiment has been described above, the present invention is not limited thereto. For example, the present invention may be applied to a configuration, illustrated in Fig. 16, in which a supply line Lb is connected between the dialysate introduction line L1 and a predetermined site of the arterial blood circuit 1 (in Fig. 16, a position between the connector 1a and the site where the blood pump 4 is provided), with clamping means K3 constituted by an electromagnetic valve provided at a halfway point of the supply line Lb. In such a configuration, the priming solution to be supplied in the priming step is the dialysate that is supplied through the supply line Lb, and is allowed to be supplied to the blood circuit when the clamping means K3 is open.

The administration step S3 may be executed as follows. In the case where the heparin H is transferred to the distal end of the arterial blood circuit 1, the blood pump 4 is normally rotated with the clamping means K2 closed, whereby the priming solution (physiological saline or dialysate) is supplied to the air-trap chamber 6 for pressure accumulation. Subsequently, the blood pump 4 is reversely rotated to cause the priming solution subjected to pressure accumulation to flow toward the arterial blood circuit 1, whereby the heparin H at the distal portion of the arterial blood circuit 1 is administered into the patient's body.

In such a case, as illustrated in Fig. 14 or 15, the control unit 9 is able to execute a pressure accumulation step (1 (s)) of accumulating the pressure between the site where the blood pump 4 is provided and the site where the clamping means K2 is provided by normally rotating the blood pump 4 with the clamping means K2 closing the flow route, and a step (2 (s)) of administering the heparin H from the distal end of the arterial blood circuit 1 by reversely rotating the blood pump 4 with the clamping means K2 kept closing the flow route. The pressure accumulation step is a step controlled by the control unit 9 in such a manner as to increase and accumulate the pressure in the air-trap chamber 6 by normally rotating the blood pump 4 with the clamping means K1 and the clamping means K2 closed but the clamping means K3 open. The pressure accumulation step enables the storing and securing of a predetermined amount of priming solution to be used in administering the heparin H from the distal end of the arterial blood circuit 1. It is preferable to provide a pressure sensor to the air-trap chamber 6 so as to detect and monitor the pressure during the pressure accumulation.

After the heparin H is transferred to only the distal portion of the arterial blood circuit 1 or only the distal portion of the venous blood circuit 2 in the transfer step, the blood pump 4 may be caused to undergo either normal rotation or reverse rotation in the administration step S3 to administer the heparin H at the distal portion of the arterial blood circuit 1 or the heparin H at the distal portion of the venous blood circuit 2 to the patient. Such an operation reduces the duration of the administration step S3.

In the case where the heparin H is transferred to both the arterial blood circuit 1 and the venous blood circuit 2, the administration step S3 according to the present embodiment is executed such that the heparin H is administered to the patient first from the distal end of the venous blood circuit 2 and then from the distal end of the arterial blood circuit 1. Alternatively, the heparin H may be administered to the patient first from the distal end of the arterial blood circuit 1 and then from the distal end of the venous blood circuit 2, or alternately from the distal end of the arterial blood circuit 1 and from the distal end of the venous blood circuit 2.

According to a first aspect of the present invention, there is provided a blood purification apparatus configured to purify a patient's blood by causing the blood to extracorporeally circulate through a blood circuit and a blood purifier, the blood circuit including an arterial blood circuit 1 and a venous blood circuit 2. The blood purification apparatus includes a blood pump 4 configured to cause the blood in the blood circuit to flow for extracorporeal circulation, an infusion pump 5 configured to infuse an anticoagulant into a predetermined site, and a control unit 9 configured to control the blood pump 4 and the infusion pump 5. The control unit 9 is configured to execute a process for activating the infusion pump 5 by supplying a driving signal to the infusion pump 5 in a state where the blood circuit is filled with a priming solution; causing the blood pump 4 to undergo a first operation by supplying a first driving signal to the blood pump 4 in a state where the anticoagulant is infused into the blood circuit by the activation of the infusion pump 5; and causing the blood pump 4 to undergo a second operation by supplying a second driving signal to the blood pump 4 in a state where the anticoagulant is transferred to a distal end of the arterial blood circuit 1 and/or a distal end of the venous blood circuit 2 by the first operation of the blood pump and where the patient is punctured with the distal end of the arterial blood circuit 1 and the distal end of the venous blood circuit 2. Such an aspect produces an effect of reducing the amount of priming solution (physiological saline) to be administered to the patient together with the anticoagulant before the blood purification treatment.

According to a second aspect of the present invention, the blood purification apparatus according to the first aspect further includes a first electromagnetic valve (clamping means K1) configured to open and close an end portion of the arterial blood circuit 1, a second electromagnetic valve (clamping means K2) configured to open and close an end portion of the venous blood circuit 2, and a third electromagnetic valve (clamping means K3) configured to open and close a supply line through which dialysate is to be supplied. The control unit 9 is configured to execute a process for opening the first electromagnetic valve and the second electromagnetic valve before the anticoagulant is transferred, closing the first electromagnetic valve and the second electromagnetic valve after the anticoagulant is transferred to the distal end of the arterial blood circuit 1 and/or the distal end of the venous blood circuit 2, and closing the third electromagnetic valve. Such an aspect produces an effect that controlling the electromagnetic valves enables the administration of the anticoagulant to the patient before the blood purification treatment.

According to a third aspect of the present invention, in the blood purification apparatus according to the second aspect, in a state where the anticoagulant is transferred to the distal end of the venous blood circuit 2 by the first operation of the blood pump 4 and where the patient is punctured with the distal end of the arterial blood circuit 1 and the distal end of the venous blood circuit 2, the second electromagnetic valve (clamping means K2) and the third electromagnetic valve (clamping means K3) are opened and the first electromagnetic valve (clamping means K1) is closed. Such an aspect produces an effect that controlling the electromagnetic valves enables the administration of the anticoagulant from the distal end of the venous blood circuit 2.

According to a fourth aspect of the present invention, in the blood purification apparatus according to the second aspect, in a state where the anticoagulant is transferred to the distal end of the arterial blood circuit 1 by the first operation of the blood pump 4 and where the patient is punctured with the distal end of the arterial blood circuit 1 and the distal end of the venous blood circuit 2, the first electromagnetic valve (clamping means K1) and the third electromagnetic valve (clamping means K3) are opened as appropriate with the second electromagnetic valve (clamping means K2) closed. Such an aspect produces an effect that controlling the electromagnetic valves enables the administration of the anticoagulant from the distal end of the arterial blood circuit 1.

According to a fifth aspect of the present invention, in the blood purification apparatus according to the fourth aspect, a pressure accumulation step of accumulating pressure and a step of reversely rotating the blood pump 4 are executed repeatedly, the pressure accumulation step being executed by normally rotating the blood pump 4 with the first electromagnetic valve (clamping means K1) and the second electromagnetic valve (clamping means K2) closed and the third electromagnetic valve (clamping means K3) open, the step of reversely rotating the blood pump being executed with the first electromagnetic valve (clamping means K1) open and the second electromagnetic valve (clamping means K2) and the third electromagnetic valve (clamping means K3) closed. Such an aspect enables the administration of the anticoagulant through pressure accumulation.

According to a sixth aspect of the present invention, in the first aspect, the control unit 9 is configured to execute a process for standing by until a predetermined time elapses after the second operation of the blood pump 4. Such an aspect enables standing by for a predetermined time after the administration of the anticoagulant.

According to a seventh aspect of the present invention, in the blood purification apparatus according to the first aspect, in a state where the distal end of the arterial blood circuit and the distal end of the venous blood circuit are connected to each other to form a closed circuit, where the closed circuit is filled with the priming solution, and where the anticoagulant is infused into the blood circuit by the activation of the infusion pump, the control unit causes the blood pump to undergo the first operation. Such an aspect enables the execution of the first operation while the closed circuit formed in the priming step is maintained.

According to an eighth aspect of the present invention, there is provided a processing method to be executed by a control unit 9 included in a blood purification apparatus in which the control unit 9 is configured to control a blood pump 4 and an infusion pump 5, the blood pump 4 being configured to cause blood in a blood circuit to flow for extracorporeal circulation, the infusion pump 5 being configured to infuse an anticoagulant into a predetermined site. The processing method includes a step of activating the infusion pump 5 by supplying a driving signal to the infusion pump 5 in a state where the blood circuit is filled with a priming solution, a step of causing the blood pump 4 to undergo a first operation by supplying a first driving signal to the blood pump 4 in a state where the anticoagulant is infused into the blood circuit by the activation of the infusion pump 5, and a step of causing the blood pump 4 to undergo a second operation by supplying a second driving signal to the blood pump 4 in a state where the anticoagulant is transferred to a distal end of the arterial blood circuit 1 and/or a distal end of the venous blood circuit 2 by the first operation of the blood pump 4 and where a patient is punctured with the distal end of the arterial blood circuit 1 and the distal end of the venous blood circuit 2. Such an aspect produces an effect of reducing the amount of priming solution (physiological saline) to be administered to the patient together with the anticoagulant before the blood purification treatment.

According to a ninth aspect of the present invention, there is provided a program for a control unit 9 included in a blood purification apparatus in which the control unit 9 is configured to control a blood pump 4 and an infusion pump 5, the blood pump 4 being configured to cause blood in a blood circuit to flow for extracorporeal circulation, the infusion pump 5 being configured to infuse an anticoagulant into a predetermined site. The program is configured to cause the control unit 9 to activate the infusion pump 5 by supplying a driving signal to the infusion pump 5 in a state where the blood circuit is filled with a priming solution, cause the blood pump 4 to undergo a first operation by supplying a first driving signal to the blood pump 4 in a state where the anticoagulant is infused into the blood circuit by the activation of the infusion pump 5, and supply a second driving signal to the blood pump 4 in a state where the anticoagulant is transferred to a distal end of the arterial blood circuit 1 and/or a distal end of the venous blood circuit 2 by the first operation of the blood pump 4 and where a patient is punctured with the distal end of the arterial blood circuit 1 and the distal end of the venous blood circuit 2. Such an aspect produces an effect of reducing the amount of priming solution (physiological saline) to be administered to the patient together with the anticoagulant before the blood purification treatment.

A storage medium may store a program for a control unit 9 included in a blood purification apparatus in which the control unit 9 is configured to control a blood pump 4 and an infusion pump 5, the blood pump 4 being configured to cause blood in a blood circuit to flow for extracorporeal circulation, the infusion pump 5 being configured to infuse an anticoagulant into a predetermined site, the program being configured to cause the control unit 9 to activate the infusion pump 5 by supplying a driving signal to the infusion pump 5 in a state where the blood circuit is filled with a priming solution; cause the blood pump 4 to undergo a first operation by supplying a first driving signal to the blood pump 4 in a state where the anticoagulant is infused into the blood circuit by the activation of the infusion pump 5; and supply a second driving signal to the blood pump 4 in a state where the anticoagulant is transferred to a distal end of an arterial blood circuit 1 and/or a distal end of a venous blood circuit 2 by the first operation of the blood pump 4 and where a patient is punctured with the distal end of the arterial blood circuit 1 and the distal end of the venous blood circuit 2.

### Industrial Applicability

The present invention is also applicable to any blood purification apparatus having a different appearance, additional functions, or the like, as long as the blood purification apparatus is equivalent to that intended by the present invention.

### Reference Signs List

- 1: arterial blood circuit
- 1a: connector
- 2: venous blood circuit
- 2a: connector
- 3: dialyzer (blood purifier)
- 4: blood pump
- 5: infusion pump
- 6: air-trap chamber
- 7: container
- 8: dialysis device
- 9: control unit
- M: syringe
- H: heparin (anticoagulant)
- K1 to K3: clamping means

## Claims

1. A blood purification apparatus configured to purify a patient's blood by causing the blood to extracorporeally circulate through a blood circuit and a blood purifier, the blood circuit including an arterial blood circuit and a venous blood circuit, the blood purification apparatus comprising:
a blood pump configured to cause the blood in the blood circuit to flow for extracorporeal circulation;
an infusion pump configured to infuse an anticoagulant into a predetermined site; and
a control unit configured to control the blood pump and the infusion pump,
wherein the control unit is configured to
activate the infusion pump by supplying a driving signal to the infusion pump in a state where the blood circuit is filled with a priming solution; cause the blood pump to undergo a first operation by supplying a first driving signal to the blood pump in a state where the anticoagulant is infused into the blood circuit by the activation of the infusion pump; and supply a second driving signal to the blood pump in a state where the anticoagulant is transferred to a distal end of the arterial blood circuit and/or a distal end of the venous blood circuit by the first operation of the blood pump and where the patient is punctured with the distal end of the arterial blood circuit and the distal end of the venous blood circuit.

2. The blood purification apparatus according to claim 1, further comprising:
a first electromagnetic valve configured to open and close an end portion of the arterial blood circuit;
a second electromagnetic valve configured to open and close an end portion of the venous blood circuit; and
a third electromagnetic valve configured to open and close a supply line through which dialysate is to be supplied,
wherein the control unit is configured to
execute a process for opening the first electromagnetic valve and the second electromagnetic valve before the anticoagulant is transferred, closing the first electromagnetic valve and the second electromagnetic valve after the anticoagulant is transferred to the distal end of the arterial blood circuit and/or the distal end of the venous blood circuit, and closing the third electromagnetic valve.

3. The blood purification apparatus according to claim 2, wherein in a state where the anticoagulant is transferred to the distal end of the venous blood circuit by the first operation of the blood pump and where the patient is punctured with the distal end of the arterial blood circuit and the distal end of the venous blood circuit, the second electromagnetic valve and the third electromagnetic valve are opened and the first electromagnetic valve is closed.

4. The blood purification apparatus according to claim 2, wherein in a state where the anticoagulant is transferred to the distal end of the arterial blood circuit by the first operation of the blood pump and where the patient is punctured with the distal end of the arterial blood circuit and the distal end of the venous blood circuit, the first electromagnetic valve and the third electromagnetic valve are opened as appropriate with the second electromagnetic valve closed.

5. The blood purification apparatus according to claim 4, wherein a pressure accumulation step of accumulating pressure and a step of reversely rotating the blood pump are executed repeatedly, the pressure accumulation step being executed by normally rotating the blood pump with the first electromagnetic valve and the second electromagnetic valve closed and the third electromagnetic valve open, the step of reversely rotating the blood pump being executed with the first electromagnetic valve open and the second electromagnetic valve and the third electromagnetic valve closed.

6. The blood purification apparatus according to claim 1, wherein the control unit is configured to execute a process for standing by until a predetermined time elapses after a second operation of the blood pump.

7. The blood purification apparatus according to claim 1, wherein in a state where the distal end of the arterial blood circuit and the distal end of the venous blood circuit are connected to each other to form a closed circuit, where the closed circuit is filled with the priming solution, and where the anticoagulant is infused into the blood circuit by the activation of the infusion pump, the control unit causes the blood pump to undergo the first operation.

8. A processing method to be executed by a control unit included in a blood purification apparatus in which the control unit is configured to control a blood pump and an infusion pump, the blood pump being configured to cause blood in a blood circuit to flow for extracorporeal circulation, the infusion pump being configured to infuse an anticoagulant into a predetermined site, the processing method comprising:
a step of activating the infusion pump by supplying a driving signal to the infusion pump in a state where the blood circuit is filled with a priming solution;
a step of causing the blood pump to undergo a first operation by supplying a first driving signal to the blood pump in a state where the anticoagulant is infused into the blood circuit by the activation of the infusion pump; and
a step of causing the blood pump to undergo a second operation by supplying a second driving signal to the blood pump in a state where the anticoagulant is transferred to a distal end of an arterial blood circuit and/or a distal end of a venous blood circuit by the first operation of the blood pump and where a patient is punctured with the distal end of the arterial blood circuit and the distal end of the venous blood circuit.

9. A program for a control unit included in a blood purification apparatus in which the control unit is configured to control a blood pump and an infusion pump, the blood pump being configured to cause blood in a blood circuit to flow for extracorporeal circulation, the infusion pump being configured to infuse an anticoagulant into a predetermined site, the program being configured to cause the control unit to activate the infusion pump by supplying a driving signal to the infusion pump in a state where the blood circuit is filled with a priming solution; cause the blood pump to undergo a first operation by supplying a first driving signal to the blood pump in a state where the anticoagulant is infused into the blood circuit by the activation of the infusion pump; and supply a second driving signal to the blood pump in a state where the anticoagulant is transferred to a distal end of an arterial blood circuit and/or a distal end of a venous blood circuit by the first operation of the blood pump and where a patient is punctured with the distal end of the arterial blood circuit and the distal end of the venous blood circuit.
